# EUROPEAN PATENT SPECIFICATION

(11) **EP 2 706 905 B1**
(45) Date of publication and mention of the grant of the patent: **24.10.2018**
(21) Application number: 12724179.2
(22) Date of filing: 23.04.2012
(51) Int. Cl.: A61B 5/00, G01N 21/17, G01N 29/24

(54) **SUBJECT INFORMATION OBTAINING APPARATUS AND SUBJECT INFORMATION OBTAINING METHOD**
VORRICHTUNG ZUM ERHALT VON PERSONENINFORMATIONEN UND VERFAHREN ZUM ERHALT VON PERSONENINFORMATIONEN
APPAREIL D'OBTENTION D'INFORMATIONS DE SUJET ET PROCÉDÉ D'OBTENTION D'INFORMATIONS DE SUJET

(30) Priority: 12.05.2011 JP 2011107254; 23.03.2012 JP 2012067575
(43) Date of publication of application: 19.03.2014
(73) Proprietor: Canon Kabushiki Kaisha, Tokyo 146-8501 (JP)
(72) Inventor: TOKITA, Toshinobu, Tokyo 146-8501 (JP)
(74) Representative: TBK
(86) International application number: PCT/JP2012/061501
(87) International publication number: WO 2012/153665

(56) References cited:
- US-A1- 2005 187 471
- US-A1- 2007 015 978
- US-A1- 2009 066 949

## Description

### Technical Field

The present invention relates to a subject information obtaining apparatus and a subject information obtaining method. In particular, the invention relates to a subject information obtaining apparatus that irradiates a subject with pulsed light and receives an acoustic wave generated in the subject to obtain internal subject information and a subject information obtaining method.

### Background Art

A photoacoustic imaging such as a photoacoustic tomography (hereinafter, which will be referred to as PAT) attracts attention as a method of specifically imaging a generated vascularization caused by cancer. The PAT is a technology of illuminating pulsed light (near-infrared ray or the like) on a subject such as a living body and receiving a photoacoustic wave generated from the inside of the living body to carry out imaging.

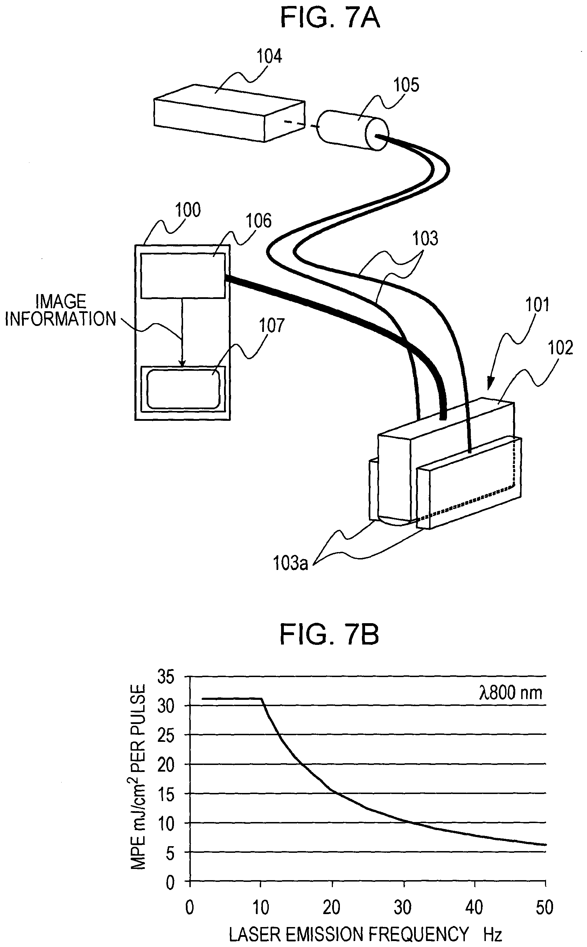

NPL 1 discloses a hand-held type apparatus using the photoacoustic imaging technology. Fig. 7A illustrates a schematic diagram of the hand-held type apparatus described in NPL 1. In Fig. 7A, in a photoacoustic probe 101, a receiver 102 configured to receive a photoacoustic wave is sandwiched and fixed by outgoing terminals 103a of bundle fibers 103. Pulsed light generated in a light source 104 enters incoming terminals of the bundle fibers 103 via an illumination optical system 105, and a subject (not illustrated) is irradiated with the pulsed light from the outgoing terminals 103a of the bundle fibers 103. The receiver 102 receives the photoacoustic wave generated from the inside of the subject to be converted to a reception signal. Then, a processing apparatus 106 of an ultrasound apparatus 100 performs an amplification and digitalization of the reception signal and thereafter performs an image reconstruction. The processing apparatus 106 outputs generated image data to a monitor 107 and displays a photoacoustic image.

### Citation List

### Non Patent Literature

NPL 1 Photons Plus Ultrasound: Imaging and Sensing 2009, Proc. of SPIE vol. 7177, 2009

### Patent Literature

Document US 2005/187471 A1 discloses a non-invasive subject-information imaging apparatus including a light generating unit which generates light containing a specific wavelength component, a light irradiation unit which radiates the generated light into a subject, a waveguide unit which guides the light from the light generating unit to the irradiation unit, a plurality of two-dimensionally arrayed electroacoustic transducer elements, a transmission/reception unit which transmits ultrasonic waves to the subject by driving the electroacoustic transducer elements, and generates a reception signal from electrical signals converted by electroacoustic transducer elements, and a signal processing unit which generates volume data about a living body function by processing a reception signal corresponding to acoustic waves generated in the subject by light irradiation, and generates volume data about a tissue morphology by processing a reception signal corresponding to echoes generated in the subject upon transmission of the ultrasonic waves.

Document US 2009/066949 A1 discloses a measurement method of measuring a spectroscopic characteristic inside of a scattering medium including a first step of measuring the spectroscopic characteristic of the scattering medium by using diffuse optical tomography by irradiating light into the scattering medium, a second step of measuring the spectroscopic characteristic of the scattering medium by using acousto-optical tomography or photo acoustic tomography by irradiating light into the scattering medium, and a third step of making an assumption of a distribution of the spectroscopic characteristic inside of the scattering medium and of changing the assumption such that a difference between a predicted value of the spectroscopic characteristic derived from the assumption and a measured value obtained in the first step can fall upon a permissible range.

### Summary of Invention

In the apparatus using the photoacoustic imaging technology, to improve contrast, an SNR (signal-to-noise ratio) of the reception signal is preferably improved. For that reason, it is conceivable to reduce noise by increasing the number of times when the reception signal is obtained and performing averaging of the reception signals. However, if the number of times when the reception signal is obtained is simply increased, a period of time for obtaining the reception signals accordingly extends. When the reception signal obtaining period extends, a positional shift or the like caused by relative movements of the subject and the photoacoustic probe may occur, and an image performance may be decreased. For that reason, it is conceivable to increase a laser emission frequency of the pulsed light.

However, as illustrated in Fig. 7B, Japanese Industrial Standards (JIS) C6802 specifies a maximum permissible exposure (MPE) against a skin. According to this specification, the MPE becomes maximum when the laser emission frequency is approximately 10 Hz or lower. If the laser emission frequency is set to be higher than 10 Hz, the exposure value is to be decreased in inverse proportion. Fig. 7B illustrates a result of a calculation while an exposure period is set as 10 seconds or longer and a wavelength is set as 800 nm. With this setting, in a case where an illumination area of the pulsed light is constant, while following an initial acoustic pressure p of the photoacoustic wave = Γµaφ (Γ: Grueneisen coefficient, µa: absorption coefficient, φ: light quantity), the light quantity φ to a tissue inside the subject (optical absorbent) is decreased in inverse proportion, and the initial acoustic pressure p of the photoacoustic wave is also decreased in inverse proportion. For example, in a case where the laser emission frequency of the pulsed light to the subject is changed from 10 Hz to 20 Hz, an illumination density (illumination light quantity per unit area) is to be halved. Instead of the noise reduction through the averaging, the original reception acoustic pressure is decreased. In the subject, since the light attenuates in an exponential manner, in particular, the light hardly reaches a deep part of the subject. As a result, an effect of the improvement in the SNR is not obtained.

The present invention has been made in view of the above-mentioned circumstances, and according to an aspect of the present invention, a period of time for obtaining reception signals is to be shortened to improve the signal-to-noise ratio.

According to an aspect of the present invention, there is provided a subject information obtaining apparatus as defined in claim 1.

According to another aspect of the present invention, there is provided a subject information obtaining method as defined in claim 12.

According to the aspects of the present invention, it is possible to improve the SNR by increasing the number of times when the reception signal is obtained, and further, it is possible to shorten the reception signal obtaining period.

### Brief Description of Drawings

Fig. 1 is a schematic diagram for describing an apparatus configuration according to a first exemplary embodiment of the present invention.
Figs. 2A and 2B are explanatory diagrams for describing a switching timing for optical paths according to the first exemplary embodiment of the present invention.
Figs. 3A and 3B are explanatory diagrams for describing a switching method of the optical paths according to the first exemplary embodiment of the present invention.
Figs. 4A and 4B are explanatory diagrams for describing the switching method of the optical paths according to the first exemplary embodiment of the present invention.
Figs. 5A and 5B are explanatory diagrams for describing a switching timing for the optical paths according to a second exemplary embodiment of the present invention.
Fig. 6 is a schematic diagram for describing a probe configuration according to a third exemplary embodiment of the present invention.
Figs. 7A and 7B are explanatory diagrams for describing a related art technology.

### Description of Embodiments

Hereinafter, embodiments of the present invention will be described by using the drawings. According to the embodiments of the present invention, an acoustic wave is typically an ultrasound wave and includes an elastic wave called sound wave, ultrasonic wave, photoacoustic wave, or optical ultrasound wave. Also, a subject information obtaining apparatus according to the embodiments of the present invention includes an apparatus that utilizes a photoacoustic wave effect of obtaining subject information as image data by irradiating a subject with light (electromagnetic wave including visible light or infra-red ray) and receiving an acoustic wave generated in the subject through the irradiation.

The obtained subject information includes a characteristic distribution such as a sound pressure distribution of the acoustic wave generated through the light irradiation, a light energy absorbing density distribution derived from the sound pressure distribution, an absorption coefficient distribution, or a density distribution of a substance constituting tissues. The density distribution of the substance is, for example, an oxygen saturation distribution, an oxidation-reduction hemoglobin estimation distribution, or the like.

According to the embodiments of the present invention, pulsed light generated from a light source is propagated to one irradiation unit, and an acoustic wave from a subject is received by a receiver. In the next light emission, pulsed light is illuminated from a different irradiation unit, and the acoustic wave is received by the receiver. In this manner, according to the embodiments of the present invention, plural irradiation units for pulsed light are provided, and the subject is not irradiated with the pulsed light continuously from one irradiation unit. Herein, "the subject is not irradiated with the pulsed light continuously" according to the embodiments of the present invention means that when the subject is irradiated once with the pulsed light from a certain irradiation unit, the subject is next irradiated with the pulsed light from a different irradiation unit. In other words, the pulsed light is not illuminated from the same irradiation unit by two times in succession.

With the above-mentioned configuration, the light irradiation is carried out at a low frequency at a position of a subject surface (skin) actually irradiated with the pulsed light. However, inside the subject, since the light diffuses, an area is created where the lights from both the irradiation units reach. Therefore, for example, in a case where two irradiation units are provided, even when mutually different areas on the subject surface are alternately irradiated with, for example, the pulsed lights from the two irradiation units at a frequency of 10 Hz, inside the subject, an area irradiated with the pulsed light at a frequency of 20 Hz is created. For that reason, by increasing the number of times when the reception signal is obtained and carrying out an averaging processing or an integrating processing (adding processing) of the mutual reception signals, the noise components can be reduced. Also, instead of the processing of the mutual reception signals, the noise components can be reduced through a combining processing of mutual pieces of image data after an image reconstruction.

A detail thereof will be described more specifically in the following exemplary embodiments.

### First Exemplary Embodiment

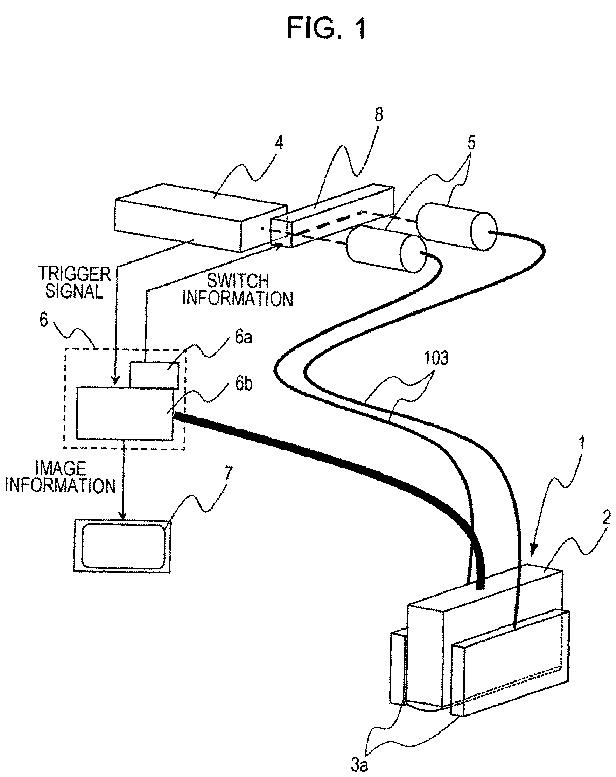

A photoacoustic apparatus that is a subject information obtaining apparatus according to a first exemplary embodiment will be described by using Fig. 1. The subject information obtaining apparatus according to the exemplary embodiment of the present invention is at least provided with a light source 4, a photoacoustic probe 1, and a processing apparatus 6.

The light source 4 generates pulsed light of near-infrared ray or the like. For the light source 4, a laser with which a large output can be obtained is preferably used, but a light emitting diode or the like can also be used instead of the laser. Preferably, an Nd:YAG laser, an alexandrite laser, or a Ti:sa laser or an OPO laser using an Nd:YAG laser beam as exciting light is used. In addition to the above, various lasers such as a solid laser, a gas laser, a dye laser, and a semiconductor laser can be used as the laser. For a wavelength of the generated light, a particular wavelength may be selected depending on a component of a measurement object (for example, hemoglobin) among lights in a range of 500 nm or higher and 1300 nm or lower.

According to the present embodiment, a beam diameter of the pulsed light generated in the light source 4 is shaped by a pulse optical system 5 that is an optical member and enters a bundle fiber 3 that is also an optical member. The bundle fiber 3 is connected to the photoacoustic probe 1.

The photoacoustic probe 1 is provided with a receiver 2 configured to receive an acoustic wave generated from the subject and converts the acoustic wave into a reception signal (electric signal) and the outgoing terminals 3a functioning as irradiation unit configured to irradiate the subject with the pulsed light. According to the present embodiment, the two outgoing terminals 3a functioning as two irradiation units (a first irradiation unit and a second irradiation unit) are provided to be symmetrical to each other with respect to the receiver 2 while sandwiching the receiver 2. The outgoing terminals 3a are outgoing terminals of the bundle fiber 3, and the light is propagated to the outgoing terminals 3a by the bundle fiber 3.

According to the exemplary embodiments of the present invention, the outgoing terminals 3a of the bundle fiber 3 may be set as the irradiation units, and the subject may be directly irradiated with the light from the outgoing terminals 3a as described above, but an arbitrary optical member such as a diffused plate may be provided. In this case, the diffused plate is set as the irradiation unit, and the subject is irradiated with the light from the diffused plate. In addition, instead of using the bundle fiber 3 for the relay of the pulsed light from the light source 4 to the subject, an optical member such as a mirror or a lens provided to a light-obstruction tube may be used. In this case, when the subject is directly irradiated with the light from an outgoing terminal of the light-obstruction tube, the outgoing terminal of the light-obstruction tube functions as the irradiation unit.

According to the present embodiment, the two outgoing terminals 3a are arranged on lateral faces of the receiver while sandwiching the receiver 2. The substantial total light quantity generated from the light source 4 is propagated to the respective outgoing terminals 3a of the bundle fiber 3. It is noted that the substantial total light quantity from the light source 4 described herein means a total light quantity where an attenuation or reflection of the light during the propagation or a consumption of the light due to a branching for a light quantity measurement or trigger obtainment is excluded. In other words, according to the present embodiment, the total light quantity is propagated to the outgoing terminal at one position from the light source 4 at the time of the one-time acoustic wave reception (that is, when the reception signal is obtained) without branching by using a half mirror or the like for propagating the pulsed light to the outgoing terminals at the two position.

The area (illumination area to the subject) of the outgoing terminals 3a of the bundle fiber is decided from a product of an outgoing terminal width in a longitudinal direction of the receiver 2 (in a case where plural elements are arranged in a one-dimensional manner, a width in a direction in which the elements are arranged) and an outgoing terminal width in a vertical direction thereof. In order that the illumination density is lower than or equal to the MPE specified by Japanese Industrial Standards (JIS) C6802 and also takes a highest possible value, the width in the vertical direction is set to be narrowed in accordance with the substantial total light quantity. With this configuration, the reception signal with respect to the irradiation per pulsed light becomes larger. The substantial total light quantity from the light source 4 is alternately emitted from the outgoing terminals 3a of the bundle fiber which sandwich the receiver 2. The receiver 2 receives the acoustic wave from each emission and transmits the reception signal to the processing apparatus 6.

The processing apparatus 6 is composed of a signal processing unit 6b and a control unit 6a. The signal processing unit 6b uses, as a trigger signal, an output from a photodiode (not illustrated) functioning as a photo detector configured to branch a part of the pulsed light for the measurement. When the trigger signal is input, the signal processing unit 6b causes the receiver 2 to receive the reception signal. The trigger signal is not limited to the output from the photodiode. A method of synchronizing the light emission of the light source 4 with the input trigger to the signal processing unit 6b may also be adopted.

After the signal processing unit 6b performs the amplification and the digital conversion of the reception signal, the signal processing unit 6b performs averaging of the reception signals obtained in plural times. It is however noted that the averaging may also be carried out before the amplification or before the digital conversion. In addition, for an averaging method, not only a simple arithmetic average but also an averaging method such as a geometrical average may also be used. Furthermore, the effects of the present invention can be obtained simply through the integrating processing (adding processing) of the reception signals for plural times instead of the averaging.

After that, the signal processing unit 6b performs an image reconstruction by using the averaged or integrated signal to generate image information (image data). Herein, the image data refers to a set of voxel data or pixel data, and this image data represents a characteristic distribution such as the absorption coefficient distribution or an oxygen saturation distribution in the subject. The signal processing unit 6b outputs this image data to a monitor 7 to be displayed.

Furthermore, according to the exemplary embodiments of the present invention, not only the processing such as the averaging or the integrating of the mutual reception signals but also the combining processing of the mutual image data pieces after the image reconstruction may be carried out. In other words, after the respective images are reconstructed by using the respective reception signals derived from the lights illuminated from the respective irradiation units, the respective pieces of image data may be mutually combined. The combining processing of the mutual pieces of image data refers to a processing of reducing the noise components by adding, multiplying, or averaging the mutual pieces of pixel data (or mutual pieces of voxel data) of the respective pieces of image data. To be more specific, the combining (for example, averaging) of the image data (first distribution) obtained by using the reception signal derived from the light illuminated from the first irradiation unit and the image data (second distribution) obtained by using the reception signal derived from the light illuminated from the second irradiation unit is conducted. After that, the combined (for example, averaged) image data (for example, the averaged distribution) is set as the characteristic distribution in the subject. Herein, for the combining processing of the mutual pieces of image data, the combining of mutual pieces of luminance data after various image processings such as an edge emphasis and a contrast adjustment are carried out or the combining of mutual pieces of data before being converted into the luminance data may suffice.

The control unit 6a is configured to control the illumination positions of the light to avoid continuous irradiation of the subject with the light from one irradiation unit. According to the present embodiment, the control unit 6a controls the illumination positions of the pulsed light by controlling a switching apparatus 8.

The switching apparatus 8 is configured to switch an optical path for the pulsed light from the light source 4 to change the illumination position of the pulsed light. In Fig. 1, the switching apparatus 8 is provided between the light source 4 and the pulse optical system 5 that shapes the diameter of the beam from the light source 4. The switching apparatus 8 switches the incidence onto the pulse optical system 5 on the basis of switch information that is a control signal from the control unit 6a in the processing apparatus 6. Through this switching, the pulsed light is alternately emitted from the outgoing terminals 3a provided so as to sandwich the receiver 2.

### Illumination Control on Pulsed Light

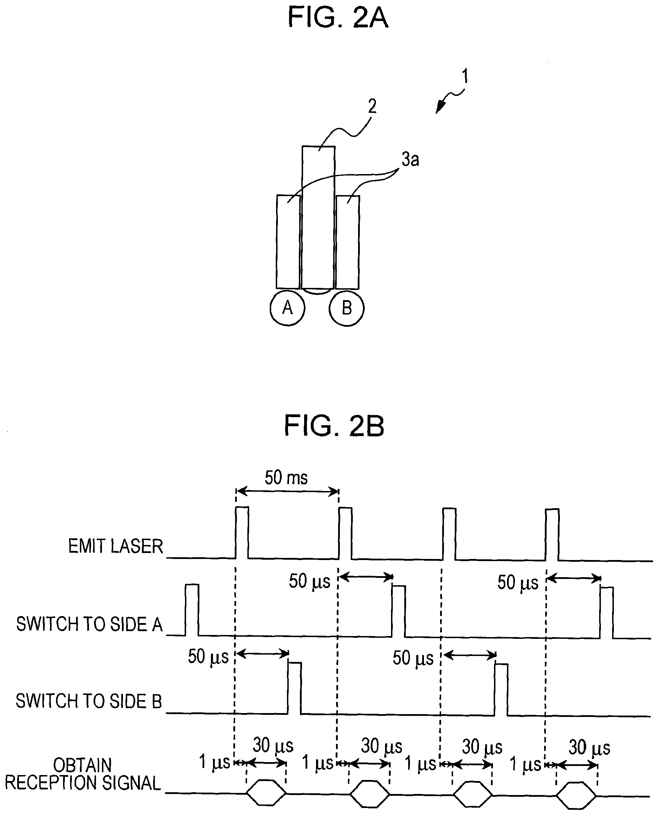

Next, a control method of the control unit 6a will be described by using a timing chart of Fig. 2B. Fig. 2A is a schematic diagram of the photoacoustic probe 1 as seen from a lateral side direction. Since the two outgoing terminals 3a are provided to be symmetrical to each other while sandwiching the receiver 2, the illumination position is divided into a side A and a side B.

According to the timing chart of Fig. 2B, 20 Hz is set as the light emission frequency of the light source 4, for example. For that reason, the light source 4 emits light at every 50 msec. First, with the switching apparatus 8, the pulsed light is illuminated at the illumination position on the side A, and the signal processing unit 6b receives the acoustic wave generated through the irradiation from the side A by using the receiver 2 and obtains the reception signal. During a period from the irradiation from the side A until the next light emission, the switching apparatus 8 performs the switch so that the pulsed light is illuminated at the illumination position on the side B. The receiver 2 receives the acoustic wave generated through the irradiation from the side B and obtains the reception signal.

Since the pulsed lights are illuminated so as to be symmetrical to each other while the receiver 2 is set as the center, the illuminated pulsed lights diffuse when a depth of the subject has at a predetermined depth of the subject a predetermined depth or deeper (for example, the depth of the subject is 3 mm or deeper). In other words, the light reaches an area within a predetermined angle range while a position immediately below the receiver 2 is set as the center line from the illumination from both the side A and the side B. At the position, the acoustic wave is generated through the illumination both on the side A and the side B. The reception signal derived from the irradiation from the side A and the reception signal derived from the irradiation from the side B have a substantially same signal waveform.

Therefore, inside the subject, the frequency at which the acoustic wave is generated can be increased twofold (20 Hz). As compared with a case in which the reception is conducted at 10 Hz, the number of signals that can be obtained in a same period of time can be doubled. For that reason, the noise components can be reduced by performing the averaging or integrating processing of the obtained reception signals. With an averaging effect at 20 Hz obtained in the same period, it is possible to reduce the noise by approximately 1/√2 as compared with a case in which an averaging effect at 10 Hz. It is of course possible to obtain the effects of the present invention also through the combining processing of the mutual pieces of image data.

In addition, even when the frequency of the illumination of the pulsed light onto the subject is increased to 20 Hz, the illumination area on the subject surface varies on every illumination (the illumination is not continuously carried out in the same area). Thus, the laser emission frequency in the same area remains the same (10 Hz). In other words, even when the light emission frequency of the light source 4 is set as 20 Hz at the substantial total light quantity from the light source 4, the pulsed light is illuminated at 10 Hz in the same area of the subject surface. Thus, the illumination can be conducted while the illumination density of approximately 30 mJ/CM² corresponding to the upper limit of the MPE with respect to the skin is maintained. Therefore, the photoacoustic wave generated from the subject and the reception signal can be obtained at an intensity at a time when the light source 4 emits the light at 10 Hz.

As described above, the number of times when the reception signal is obtained can be increased without decreasing the intensity of the reception signal, and the noise components can therefore be reduced through the effect of the averaging or integrating processing of the mutual reception signals or the combining processing of the mutual pieces of image data. It is noted that the description has been given while the light emission frequency of the light source 4 is set as 20 Hz. This configuration exemplifies an example in which the laser emission frequency can be doubled without decreasing the substantial total light quantity from the light source 4, but the embodiment is not limited to the above.

### Specific Configuration of Switching Apparatus

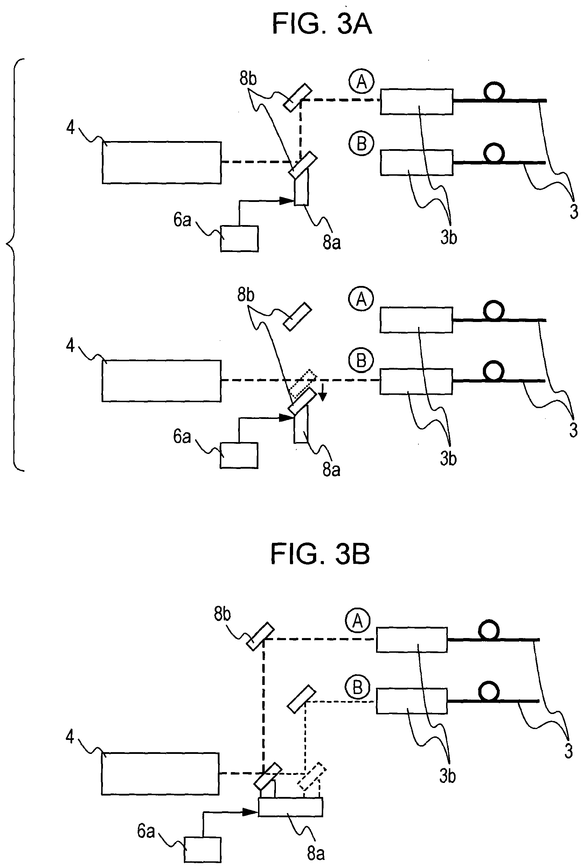

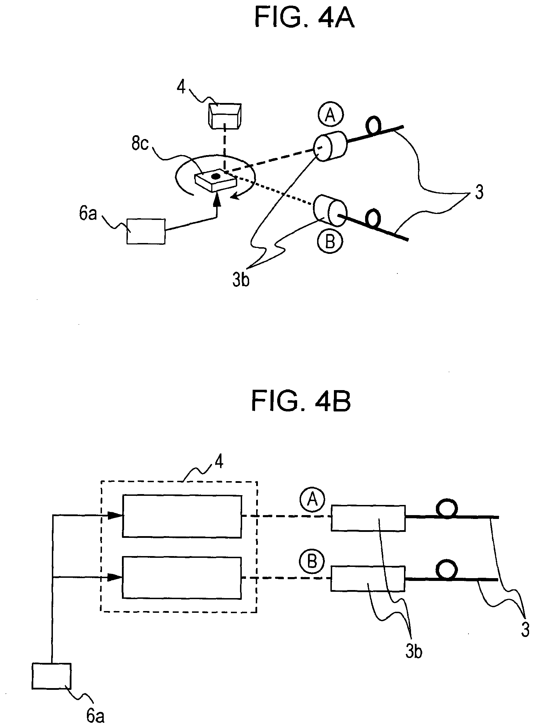

Next, by using Figs. 3A and 3B and Figs. 4A and 4B, the switching apparatus 8 will be described. To simplify the description on the switching apparatus 8, in Figs. 3A and 3B and Figs. 4A and 4B, the pulse optical system 5 is not illustrated.

The switching apparatus 8 of Fig. 3A is composed of a mirror 8b that switches the optical path between the side A and the side B (between the first irradiation unit side and the second irradiation unit side) and an actuator 8a that drives the mirror 8b. In order that the pulsed light enters an incoming terminal 3b of the bundle fiber on the side A, a drive is conducted to cause the mirror 8b to reflect the light (see an illustration at an upper part of Fig. 3A). Also, in order that the pulsed light enters an incoming terminal 3b on the side B, a drive is conducted to cause the mirror 8b not to reflect the light (see an illustration at a lower part of Fig. 3A). In either drive, the actuator 8a is driven by control signals from the control unit 6a. In addition, a switching based on a combination of the actuator 8a and the mirror 8b may correspond to a switching between the side A and the side B by changing a position of the mirror 8b on the actuator 8a as illustrated in Fig. 3B.

Furthermore, the switching apparatus 8 of Fig. 4A employs a polygonal mirror 8c instead of the actuator 8a and the mirror 8b. The polygonal mirror 8c rotates in synchronism with the light emission frequency of the light source 4 and is adjusted so that the light enters the respective incoming terminals 3b of the bundle fibers on the side A and the side B.

In addition, the switching apparatus 8 can employ not only the configurations described in Figs. 3A and 3B and Figs. 4A and 4B but also a galvano-mirror, an acousto-optical deflector (AOD), or the like.

Furthermore, according to the exemplary embodiments of the present invention, it is also possible to switch the illumination positions without using the switching apparatus 8. To be more specific, as illustrated in Fig. 4B, the light source 4 composed of a first light source and a second light source is used. On the basis of the control signals from the control unit 6a, the light source 4 controls the light emission timings of the first light source and the second light source so that the illumination positions can be switched.

As described above, according to the present embodiment, the number of times when the reception signal is obtained can be increased without decreasing the intensity of the reception signal, and the noise components can be reduced through the effect of the averaging or integrating processing of the mutual reception signals or the combining processing of the mutual pieces of image data. As a result, the SNR is improved, and the contrast is improved after the imaging. Thus, a legibility and a clinical diagnostic performance are improved.

### Second Exemplary Embodiment

According to the first exemplary embodiment, the mode has been described in which the outgoing terminals 3a of the bundle fiber are provided one by one at the positions corresponding to the illumination areas of the pulsed light while sandwiching the receiver 2, and the illumination is carried out alternately. According to a second exemplary embodiment, a mode will be described in which still more outgoing terminals functioning as the irradiation units are provided. A configuration other than the number of the optical paths for the pulsed light from the light source and the structure of the photoacoustic probe is the same as the first exemplary embodiment, and a description thereof will be omitted.

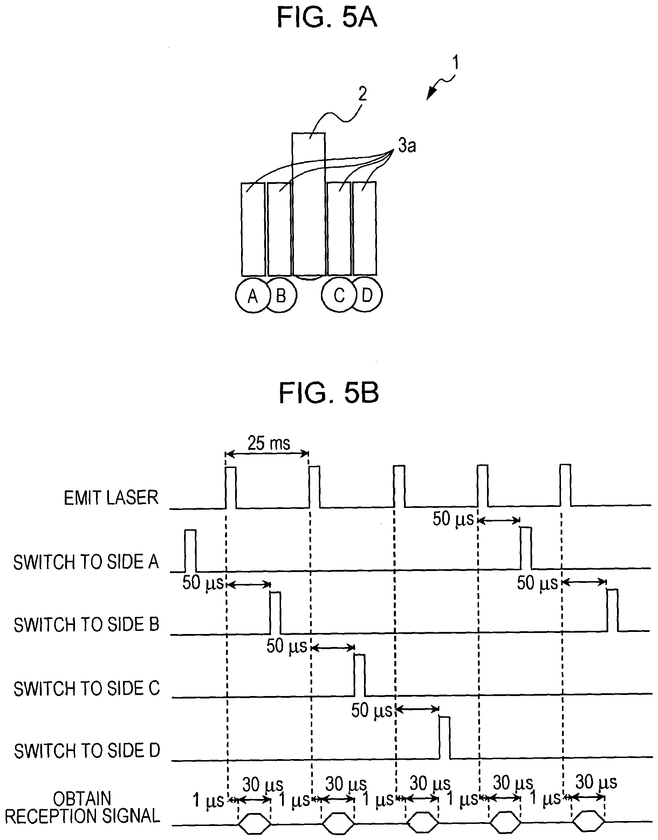

Fig. 5A is a schematic diagram of the photoacoustic probe 1 as seen from the lateral side direction according to the present embodiment. The photoacoustic probe 1 is provided with four outgoing terminals 3a (a first irradiation unit, a second irradiation unit, a third irradiation unit, and a fourth irradiation unit), the illumination positions are divided for two positions each including a set of a side A and a side B and a set of a side C and a side D while sandwiching the receiver 2. The substantial total light quantity from the light source 4 is output from each of the outgoing terminals 3a of the bundle fiber. According to a timing chart of Fig. 5B, 40 Hz is set as the light emission frequency of the light source 4, for example. That is, the light source 4 emits light at every 25 msec.

In Fig. 5B, first, the pulsed light is caused to enter the side A by the switching apparatus 8, and the receiver 2 obtains the reception signal of the acoustic wave derived from the irradiation from the side A. During a period from this irradiation with the pulsed light until the next pulsed light emission, the switching apparatus 8 performs the switch so that the pulsed light enters the side B. After that, the receiver 2 obtains the reception signal of the acoustic wave derived from the irradiation from the side B. The above-mentioned flow is repeated on the side C and the side D. It is noted that the description has been given while the light emission frequency of the light source 4 is set as 40 Hz, but the embodiment is not limited to the above.

At this time, the illumination positions on the side A and the side D on an outer side are symmetrical to each other while sandwiching the receiver 2, and also the illumination positions on the side B and the side C are symmetrical to each other while sandwiching the receiver 2. Therefore, the illuminated lights diffuse when a depth of the subject has at a predetermined depth of the subject a predetermined depth or deeper (in particular, the depth of the subject is 3 mm or deeper). Thus, the mutual reception signals derived from the pulsed lights that are illuminated from the outer side (the side A and the side D) have substantially a same signal waveform. Similarly, the mutual reception signals derived from the pulsed lights that are illuminated from the inner side (the side B and the side C) also have substantially a same signal waveform.

However, the illumination positions on the inner side and the illumination positions on the outer side are not symmetrical to each other while sandwiching the receiver 2. Thus, the reception signal derived from the irradiation from the inner side and the reception signal derived from the irradiation from the outer side have different signal waveforms. For example, at the position in the subject below the receptor, the quantity of the reaching light is decreased in the irradiation from the outer side as compared with the irradiation from the inner side. With this difference in the light quantity, a difference occurs in the sound pressure of the received acoustic wave. Thus, the signal waveforms of the reception signals are different from each other. In other words, the reception signal derived from the irradiation from the outer side has a lower amplitude (intensity) than the reception signal derived from the irradiation from the inner side.

For that reason, the signal processing unit 6b preferably conducts a correction on the reception signal between the irradiation from the outer side and the irradiation from the inner side. To be more specific, the reception signal derived from the irradiation from the inner side may be multiplied with a gain for the decrease to adjust the amplitude. With this configuration, even when the pulsed light is illuminated from any of the illumination positions from the side A to the side D, the reception signals become substantially the same signals.

It is noted that the gain depends on the depth of the subject and furthermore may be analytically decided in accordance with distances between the respective illumination positions from the outside and the inner side and the receiver 2 or the subject tissues. For the analysis, a light diffusion equation and the initial sound pressure p of the acoustic wave = Γµaφ (Γ: Grueneisen coefficient, µa: absorption coefficient, φ: light quantity) can be used. Alternatively, the gain may be experimentally decided by using a phantom where an optical characteristic is already identified.

According to the present embodiment, the order of the illumination of the pulsed light is not limited to the order illustrated in Fig. 5B, and it suffices if the illumination is not carried out continuously on the same illumination position at least. Also, in Fig. 5A, the outgoing terminals 3a of the bundle fibers corresponding to the irradiation units are provided for two locations each while sandwiching the receiver 2, but the number of the outgoing terminals 3a may be further increased. In addition, with regard to the switching of the illumination positions of the pulsed light, the switching apparatus 8 or the switching method described the first exemplary embodiment by using Figs. 3A and 3B and Figs. 4A and 4B may be applied.

As described above, according to the second exemplary embodiment, the number of times when the reception signal is obtained can be further increased while the intensity of the reception signal is not decreased too much (in other words, the laser emission frequency can be further increased), and the noise components can be reduced through the effect of the averaging or integrating processing of the mutual reception signals or the combining processing of the mutual pieces of image data. As a result, the SNR is improved, and the contrast is improved after the imaging. Thus, the legibility and the clinical diagnostic performance are improved.

### Third Exemplary Embodiment

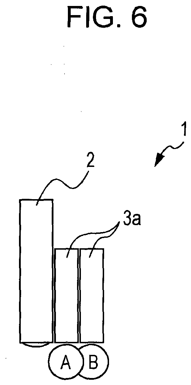

According to the first exemplary embodiment and the second exemplary embodiment, the mode has been described in which the outgoing terminals 3a of the bundle fibers functioning as the irradiation units of the pulsed light are provided so as to sandwich the receiver 2. According to a third exemplary embodiment, a mode will be described in which the outgoing terminals 3a of the plural bundle fibers are provided on one lateral face side of the receiver 2. As an example, in Fig. 6, the two outgoing terminals 3a of the bundle fibers are both provided on one side of the receiver 2. It is noted that the basis apparatus configuration and the method for the averaging or integrating processing of the mutual reception signals or the combining processing of the mutual pieces of image data have been described in the first exemplary embodiment and the second exemplary embodiment, and a description thereof will be omitted.

In Fig. 6, as described in the second exemplary embodiment, the distance from the receiver 2 varies on the side A and the side B of the illumination areas of the pulsed light, and the intensity of the acoustic wave received by the receiver 2 varies. For that reason, the reception signal may be multiplied with a gain decided analytically and/or experimentally.

Also, in combination with the second exemplary embodiment, different numbers of the outgoing terminals 3a of the bundle fibers corresponding to the irradiation units of the pulsed light may be provided, for example, at two locations on one side and three locations on the other side.

As described above, according to the third exemplary embodiment, it is possible to arbitrarily set the locations of the outgoing terminals of the pulsed light which are provided next to the receiver 2. For that reason, it is facilitated to set a form of the photoacoustic probe 1 easier for an operator to hold.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

### Reference Signs List

- 1: photoacoustic probe
- 2: receiver
- 3: bundle fiber
- 3a: outgoing terminal
- 3b: incoming terminal
- 4: light source
- 5: pulse optical system
- 6: processing apparatus
- 6a: control unit
- 6b: signal processing unit
- 7: monitor
- 8: switching apparatus

## Claims

1. A subject information obtaining apparatus that obtains information regarding a subject, the apparatus comprising:
a light source (4) configured to generate pulsed light;
a probe (1) including a receiver (2) configured to receive an acoustic wave generated in the subject by pulsed light generated by the light source (4) and convert the acoustic wave to an electric signal, a first irradiation unit (3a), and a second irradiation unit (3a), the first and second irradiation units (3a) configured to irradiate mutually different areas on a surface of the subject with the pulsed light generated by the light source (4);
a signal processing unit (6b) configured to obtain the information regarding the subject by using the electric signal; and
a control unit (6a) configured to control illumination positions of the pulsed light to avoid continuous irradiation of the subject with the pulsed light from each of the first irradiation unit (3a) and the second irradiation unit (3a), wherein
the control unit (6a) is configured to control the first irradiation unit (3a) and the second irradiation unit (3a) to alternately illuminate the pulsed light such that the frequency at which the acoustic wave is generated can be increased, wherein
the receiver (2) is configured to receive an acoustic wave generated in the subject having been irradiated with the pulsed light from the first irradiation unit (3a) and to convert the acoustic wave to a first electric signal, and to receive an acoustic wave generated in the subject having been irradiated with the pulsed light from the second irradiation unit (3a) and convert the acoustic wave to a second electric signal;
the control unit (6a) is configured to control timing of light irradiation from the first irradiation unit and timing of light irradiation from the second irradiation unit; and
the signal processing unit (6b) is configured to perform processing on the first electric signal and the second electric signal to obtain the information regarding the subject.

2. The subject information obtaining apparatus according to Claim 1, wherein the first and second irradiation units (3a) are arranged on lateral faces of the receiver (2).

3. The subject information obtaining apparatus according to any one of Claims 1 to 2, wherein the first and second irradiation units (3a) are arranged to be symmetrical to each other with respect to the receiver (2).

4. The subject information obtaining apparatus according to any one of Claims 1 to 3,
further comprising a switching apparatus (8) configured to switch an optical path of the pulsed light from the light source (4) between a first irradiation unit side and a second irradiation unit side,
wherein the control unit (6a) controls the illumination positions by controlling the switching apparatus (8).

5. The subject information obtaining apparatus according to Claim 1,
wherein the light source (4) is composed of a first light source that generates pulsed light propagated to the first irradiation unit (3a) and a second light source that generates the pulsed light propagated to the second irradiation unit (3a), and
wherein the control unit (6a) controls the illumination positions by controlling light emission timings of the first light source and the second light source.

6. The subject information obtaining apparatus according to any one of Claims 1 to 5, wherein the probe (1) further includes an irradiation unit configured to irradiate the subject with the pulsed light other than the first and second irradiation units.

7. The subject information obtaining apparatus according to Claim 6, wherein the signal processing unit (6b) corrects an intensity of each electric signal derived from the pulsed light illuminated from the respective irradiation units in accordance with positions of the respective irradiation units.

8. The subject information obtaining apparatus according to Claim 1, wherein the first and second irradiation units are both arranged on one lateral face of the receiver (2).

9. The subject information obtaining apparatus according to any one of Claims 1 to 8, wherein the receiver (2) configured to receive a first acoustic wave generated by the pulsed light emitted from the first irradiation unit and convert the acoustic wave to a first electric signal is same as the receiver (2) configured to receive a second acoustic wave generated by the pulsed light emitted from the second irradiation unit and convert the acoustic wave to a second electric signal.

10. The subject information obtaining apparatus according to any one of Claim 9, wherein the signal processing unit (6b) is configured to obtain a characteristic distribution in the subject by using the first electric signal and the second electric signal.

11. The subject information obtaining apparatus according to any one of Claims 1 to 10,
wherein the information regarding the subject is obtained by carrying out an averaging processing, an integrating processing, or an adding processing of mutual electric signals outputted repetitively from the receiver (2) correspondingly to irradiation of pulsed light from the first and second irradiation units(3a).

12. A subject information obtaining method that obtains information regarding a subject, the method comprising:
irradiating the subject with light generated by a light source (4) using a first irradiation unit (3a) and a second irradiation unit (3b) each configured to irradiate, with the light generated by the light source(4), a different surface area of the subject,
wherein the first irradiation unit (3a) and the second irradiation unit (3a) alternately illuminate the pulsed light such that the frequency at which an acoustic wave is generated can be increased;
causing a receiver (2), which has received an acoustic wave generated in the subject by the irradiation of the subject with the light from the first irradiation unit (3a), to output a first electric signal, and causing the receiver (2), which has received an acoustic wave generated in the subject by the irradiation of the subject with the light from the second irradiation unit (3a), to output a second electric signal;
performing control of irradiation timing with light from the first irradiation unit (3a) and irradiation timing with light from the second irradiation unit (3a); and
performing signal processing on the first electric signal and the second electric signal to obtain the information regarding the subject.

13. The subject information obtaining method according to Claim 12, wherein the method further comprises;
obtaining a characteristic distribution in the subject by using the first electric signal and the second electric signal.

## Patentansprüche

1. Subjektinformationserhaltungsvorrichtung, die eine charakteristische Verteilung in einem Subjekt erhält, wobei die Vorrichtung aufweist:
eine Lichtquelle (4), die dazu konfiguriert ist, gepulstes Licht zu erzeugen;
eine Sonde (1) mit einem Empfänger (2), der dazu konfiguriert ist, eine akustische Welle, die in dem Subjekt durch das gepulste Licht erzeugt wird, das durch die Lichtquelle (4) erzeugt wird, zu empfangen und die akustische Welle in ein elektrisches Signal umzuwandeln, einer ersten Bestrahlungseinheit (3a) und einer zweiten Bestrahlungseinheit (3a), wobei die erste und zweite Bestrahlungseinheit (3a) dazu konfiguriert sind, gegenseitig unterschiedliche Bereiche auf einer Oberfläche des Subjekts mit dem gepulsten Licht, das durch die Lichtquelle (4) erzeugt wird, zu bestrahlen;
eine Signalverarbeitungseinheit (6b), die dazu konfiguriert ist, die Informationen bezüglich des Subjekts unter Verwendung des elektrischen Signals zu erhalten; und
eine Steuerungseinheit (6a), die dazu konfiguriert ist, Beleuchtungspositionen des gepulsten Lichts zu steuern, um eine kontinuierliche Bestrahlung des Subjekts mit dem gepulsten Licht von jeder der ersten Bestrahlungseinheit (3a) und der zweiten Bestrahlungseinheit (3a) zu vermeiden, wobei
die Steuerungseinheit (6a) dazu konfiguriert ist, die erste Bestrahlungseinheit (3a) und die zweite Bestrahlungseinheit (3a) zu steuern, um das gepulste Licht abwechselnd abzustrahlen, so dass die Frequenz, mit der die akustische Welle erzeugt wird, erhöht werden kann, wobei
der Empfänger (2) dazu konfiguriert ist, eine akustische Welle, die in dem Subjekt, das mit dem gepulsten Licht von der ersten Bestrahlungseinheit (3a) bestrahlt wird, erzeugt wird, zu empfangen und die akustische Welle in ein erstes elektrisches Signal umzuwandeln, und eine akustische Welle, die in dem Subjekt, das mit dem gepulsten Licht von der zweiten Bestrahlungseinheit (3a) bestrahlt wird, erzeugt wird, zu empfangen und die akustische Welle in ein zweites elektrisches Signal umzuwandeln, und
die Signalverarbeitungseinheit (6a) dazu konfiguriert ist, einen Zeitpunkt einer Lichtbestrahlung von der ersten Bestrahlungseinheit und einen Zeitpunkt einer Lichtbestrahlung von der zweiten Bestrahlungseinheit zu steuern; und
die Signalverarbeitungseinheit (6b) dazu konfiguriert ist, eine Verarbeitung bezüglich des ersten elektrischen Signals und des zweiten elektrischen Signals durchzuführen, um die Informationen bezüglich des Subjekts zu erhalten.

2. Subjektinformationserhaltungsvorrichtung gemäß Anspruch 1, wobei die erste und zweite Bestrahlungseinheit (3a) auf seitlichen Flächen des Empfängers (2) angeordnet sind.

3. Subjektinformationserhaltungsvorrichtung gemäß einem der Ansprüche 1 bis 2, wobei die erste und zweite Bestrahlungseinheit (3a) angeordnet sind, sodass diese mit Bezug auf den Empfänger (2) zueinander symmetrisch sind.

4. Subjektinformationserhaltungsvorrichtung gemäß einem der Ansprüche 1 bis 3,
weiterhin mit einer Umschaltvorrichtung (8), die dazu konfiguriert ist, einen optischen Pfad des gepulsten Lichts von der Lichtquelle (4) zwischen einer Seite einer ersten Bestrahlungseinheit und einer Seite einer zweiten Bestrahlungseinheit umzuschalten,
wobei die Steuerungseinheit (6a) die Beleuchtungspositionen durch Steuern der Umschaltvorrichtung (8) steuert.

5. Subjektinformationserhaltungsvorrichtung gemäß Anspruch 1,
wobei die Lichtquelle (4) aus einer ersten Lichtquelle, die gepulstes Licht erzeugt, das zu der ersten Bestrahlungseinheit (3a) weitergeleitet wird, und einer zweiten Lichtquelle, die das gepulste Licht erzeugt, das an die zweite Bestrahlungseinheit (3a) weitergeleitet wird, besteht und
wobei die Steuerungseinheit (6a) die Beleuchtungspositionen durch Steuern der Lichtabstrahlungszeitpunkte der ersten Lichtquelle und der zweiten Lichtquelle steuert.

6. Subjektinformationserhaltungsvorrichtung gemäß einem der Ansprüche 1 bis 5, wobei die Sonde (1) weiterhin eine Bestrahlungseinheit aufweist, die dazu konfiguriert ist, das Subjekt mit dem gepulsten Licht zu bestrahlen, das von der ersten und zweiten Bestrahlungseinheit verschieden ist.

7. Subjektinformationserhaltungsvorrichtung gemäß Anspruch 6, wobei die Signalverarbeitungseinheit (6b) eine Intensität von jedem elektrischen Signal, das von dem gepulsten Licht hergeleitet wird, das von den entsprechenden Bestrahlungseinheiten ausgestrahlt wird, gemäß Positionen der entsprechenden Bestrahlungseinheiten korrigiert.

8. Subjektinformationserhaltungsvorrichtung gemäß Anspruch 1, wobei die erste und zweite Bestrahlungseinheit beide auf einer seitlichen Fläche des Empfängers (2) angeordnet sind.

9. Subjektinformationserhaltungsvorrichtung gemäß einem der Ansprüche 1 bis 8, wobei der Empfänger (2), der dazu konfiguriert ist, eine erste akustische Welle, die durch das gepulste Licht, das von der ersten Bestrahlungseinheit ausgesendet wird, erzeugt wird, zu empfangen und die akustische Welle in ein erstes elektrisches Signal umzuwandeln, der gleiche ist wie der Empfänger (2), der dazu konfiguriert ist, eine zweite akustische Welle, die durch das gepulste Licht, das von der zweiten Bestrahlungseinheit ausgesendet wird, erzeugt wird, zu empfangen und die akustische Welle in ein zweites elektrisches Signal umzuwandeln.

10. Subjektinformationserhaltungsvorrichtung gemäß Anspruch 9, wobei die Signalverarbeitungseinheit (6b) dazu konfiguriert ist, eine charakteristische Verteilung in dem Subjekt unter Verwendung des ersten elektrischen Signals und des zweiten elektrischen Signals zu erhalten.

11. Subjektinformationserhaltungsvorrichtung gemäß einem der Ansprüche 1 bis 10,
wobei die Informationen bezüglich des Subjekts durch Durchführen einer Mittelungsverarbeitung, einer Integrationsverarbeitung oder einer Additionsverarbeitung von gemeinsamen elektrischen Signalen, die wiederholt von dem Empfänger (2) entsprechend einer Bestrahlung von gepulstem Licht von der ersten und zweiten Bestrahlungseinheit (3a) ausgegeben werden, erhalten werden.

12. Subjektinformationserhaltungsverfahren, das Information bezüglich eines Subjekts erhält, wobei das Verfahren aufweist:
Bestrahlen des Subjekts mit Licht, das durch eine Lichtquelle (4) unter Verwendung einer ersten Bestrahlungseinheit (3a) und einer zweiten Bestrahlungseinheit (3a) erzeugt wird, die jeweils dazu konfiguriert sind, einen gegenseitig unterschiedlichen Oberflächenbereich des Subjekts mit dem Licht, das durch die Lichtquelle (4) erzeugt wird, zu bestrahlen,
wobei die erste Bestrahlungseinheit (3a) und die zweite Bestrahlungseinheit (3a) das gepulste Licht abwechselnd ausstrahlen, so dass die Frequenz, mit der die akustische Welle erzeugt wird, erhöht werden kann,
Veranlassen eines Empfängers (2), der eine akustische Welle empfangen hat, die in dem Subjekt durch die Bestrahlung des Subjekts mit dem Licht von der ersten Bestrahlungseinheit (3a) erzeugt wird, ein erstes elektrisches Signal auszugeben, und Veranlassen des Empfängers (2), der eine akustische Welle empfangen hat, die in dem Subjekt durch die Bestrahlung des Subjekts mit dem Licht von der zweiten Bestrahlungseinheit (3a) erzeugt wird, ein zweites elektrischen Signals auszugeben;
Durchführen einer Steuerung eines Bestrahlungszeitpunkts mit Licht von der ersten Bestrahlungseinheit (3a) und eines Bestrahlungszeitpunkts mit Licht von der zweiten Bestrahlungseinheit (3a); und
Durchführen einer Signalverarbeitung bezüglich des ersten elektrischen Signals und des zweiten elektrischen Signals, um die Informationen bezüglich des Subjekts zu erhalten.

13. Subjektinformationserhaltungsverfahren gemäß Anspruch 12, wobei das Verfahren weiterhin aufweist:
Erhalten einer charakteristischen Verteilung in dem Subjekt unter Verwendung des ersten elektrischen Signals und des zweiten elektrischen Signals.

## Revendications

1. Appareil d'obtention d'information de sujet qui obtient une information concernant un sujet, l'appareil comprenant :
une source de lumière (4) configurée de façon à générer une lumière puisée ;
une sonde (1) comprenant un récepteur (2) configuré pour recevoir une onde acoustique générée dans le sujet par la lumière pulsée générée par la source de lumière (4) et pour convertir l'onde acoustique en un signal électrique, une première unité d'irradiation (3a), et une deuxième unité d'irradiation (3a), les première et deuxième unités d'irradiation (3a) étant configurées pour irradier des zones mutuellement différentes sur une surface du sujet avec la lumière pulsée générée par la source de lumière (4) ;
une unité de traitement du signal (6b) configurée pour obtenir l'information concernant le sujet à l'aide du signal électrique ; et
une unité de commande (6a) configurée pour commander des positions d'illumination de la lumière pulsée de façon à éviter une irradiation continue du sujet avec la lumière pulsée venant de chacune de la première unité d'irradiation (3a) et de la deuxième unité d'irradiation (3a),
dans lequel :
l'unité de commande (6a) est configurée pour commander la première unité d'irradiation (3a) et la deuxième unité d'irradiation (3a) de façon à émettre en alternance la lumière pulsée de telle sorte que la fréquence à laquelle l'onde acoustique est générée puisse être accrue, dans lequel :
le récepteur (2) est configuré pour recevoir une onde acoustique générée dans le sujet qui a été irradié par la lumière pulsée venant de la première unité d'irradiation (3a), et pour convertir l'onde acoustique en un premier signal électrique, et pour recevoir une onde acoustique générée dans le sujet qui a été irradié par la lumière pulsée venant de la deuxième unité d'irradiation (3a), et pour convertir l'onde acoustique en un deuxième signal électrique ;
l'unité de commande (6a) est configurée pour commander le minutage d'irradiation de lumière à partir de la première unité d'irradiation et le minutage d'irradiation de lumière à partir de la deuxième unité d'irradiation ; et
l'unité de traitement du signal (6b) est configurée pour effectuer un traitement sur le premier signal électrique et le deuxième signal électrique de façon à obtenir l'information concernant le sujet.

2. Appareil d'obtention d'information de sujet selon la revendication 1, dans lequel les première et deuxième unités d'irradiation (3a) sont disposées sur des faces latérales du récepteur (2).

3. Appareil d'obtention d'information de sujet selon l'une quelconque des revendications 1 et 2, dans lequel les première et deuxième unités d'irradiation (3a) sont disposées de façon à être symétriques l'une par rapport à l'autre vis-à-vis du récepteur (2).

4. Appareil d'obtention d'information de sujet selon l'une quelconque des revendications 1 à 3,
comprenant de plus un appareil de commutation (8) configuré pour commuter un trajet optique de la lumière pulsée venant de la source de lumière (4) entre un côté de première unité d'irradiation et un côté de deuxième unité d'irradiation,
dans lequel l'unité de commande (6a) commande les positions d'illumination par la commande de l'appareil de commutation (8).

5. Appareil d'obtention d'information de sujet selon la revendication 1,
dans lequel la source de lumière (4) est constituée par une première source de lumière qui génère une lumière pulsée propagée vers la première unité d'irradiation (3a) et une deuxième source de lumière qui génère la lumière pulsée propagée vers la deuxième unité d'irradiation (3a), et
dans lequel l'unité de commande (6a) commande les positions d'illumination par la commande de minutages d'émission de lumière de la première source de lumière et de la deuxième source de lumière.

6. Appareil d'obtention d'information de sujet selon l'une quelconque des revendications 1 à 5, dans lequel la sonde (1) comprend de plus une unité d'irradiation, configurée pour irradier le sujet avec la lumière pulsée, autre que les première et deuxième unités d'irradiation.

7. Appareil d'obtention d'information de sujet selon la revendication 6, dans lequel l'unité de traitement du signal (6b) corrige une intensité de chaque signal électrique dérivé à partir de la lumière pulsée émise à partir des unités d'irradiation respectives en fonction de positions des unités d'irradiation respectives.

8. Appareil d'obtention d'information de sujet selon la revendication 1, dans lequel les première et deuxième unités d'irradiation sont toutes deux disposées sur une face latérale du récepteur (2).

9. Appareil d'obtention d'information de sujet selon l'une quelconque des revendications 1 à 8, dans lequel le récepteur (2) configuré pour recevoir une première onde acoustique générée par la lumière pulsée émise à partir de la première unité d'irradiation et convertir l'onde acoustique en un premier signal électrique est le même que le récepteur (2) configuré pour recevoir une deuxième onde acoustique générée par la lumière pulsée émise à partir de la deuxième unité d'irradiation et convertir l'onde acoustique en un deuxième signal électrique.

10. Appareil d'obtention d'information de sujet selon la revendication 9, dans lequel l'unité de traitement du signal (6b) est configurée pour obtenir une distribution de caractéristiques dans le sujet à l'aide du premier signal électrique et du deuxième signal électrique.

11. Appareil d'obtention d'information de sujet selon l'une quelconque des revendications 1 à 10,
dans lequel l'information concernant le sujet est obtenue par la réalisation d'un traitement de réalisation de moyenne, d'un traitement d'intégration, ou d'un traitement d'addition de signaux électriques mutuels délivrés en sortie de façon répétée à partir du récepteur (2) d'une façon correspondant à l'irradiation de lumière pulsée venant des première et deuxième unités d'irradiation (3a) .

12. Procédé d'obtention d'information de sujet qui obtient une information concernant un sujet, le procédé comprenant :
l'irradiation du sujet avec une lumière générée par une source de lumière (4) à l'aide d'une première unité d'irradiation (3a) et d'une deuxième unité d'irradiation (3a) chacune configurée de façon à irradier, avec la lumière générée par la source de lumière (4), une surface différente du sujet,
dans lequel la première unité d'irradiation (3a) et la deuxième unité d'irradiation (3a) illuminent en alternance la lumière pulsée de telle sorte que la fréquence à laquelle une onde acoustique est générée puisse être accrue ;
le fait de faire délivrer en sortie par un récepteur (2), qui a reçu une onde acoustique générée dans le sujet par l'irradiation du sujet par la lumière venant de la première unité d'irradiation (3a), un premier signal électrique, et le fait de faire délivrer en sortie par le récepteur (2), qui a reçu une onde acoustique générée dans le sujet par l'irradiation du sujet par la lumière venant de la deuxième unité d'irradiation (3a), un deuxième signal électrique ;
la réalisation d'une commande d'un minutage d'irradiation avec une lumière venant de la première unité d'irradiation (3a) et d'un minutage d'irradiation avec une lumière venant de la deuxième unité d'irradiation (3a) ; et la réalisation d'un traitement du signal sur le premier signal électrique et le deuxième signal électrique pour obtenir l'information concernant le sujet.

13. Procédé d'obtention d'information de sujet selon la revendication 12, dans lequel le procédé comprend de plus :
l'obtention d'une distribution de caractéristiques dans le sujet à l'aide du premier signal électrique et du deuxième signal électrique.
